# EUROPEAN PATENT APPLICATION

(11) **EP 3 967 772 A1**
(43) Date of publication of application: **16.03.2022**
(21) Application number: 20195885.7
(22) Date of filing: 14.09.2020
(51) Int. Cl.: C12Q 1/6844, G01N 33/558, C12Q 1/6883

(54) **TOOLS & METHODS USEFOOL FOR DETECTION OF LACTOSE INTOLERANCE AND USES THEREOF**

(71) Applicant: Ducrest, Percevent, 1892 Lavey (CH); Harnischberg, Franck, 1936 Verbier/Bagnes (CH)
(72) Inventor: Ducrest, Percevent, 1892 Lavey (CH); Harnischberg, Franck, 1936 Verbier/Bagnes (CH)
(74) Representative: reuteler & cie SA

(57) **Abstract**

The present invention relates to a method for the detection of lactose tolerance markers in a sample by lateral flow immunoassay, a lateral flow immunoassay device, primers and extraction buffer and uses thereof and related tools and assays useful in said method.

SEQUENCE LISTING

<110>  DUCREST, Percevent
       HARNISCHBERG, Franck

<120>  TOOLS & METHODS USEFOOL FOR DETECTION OF LACTOSE INTOLERANCE AND
       USES THEREOF

<130>  P2457EP00

<160>  11

<170>  PatentIn version 3.5

<210>  1
<211>  42
<212>  DNA
<213>  Artificial sequence

<220>
<223>  unlabeled Forward Inner Primer (FIP) sequence used for LAMP of T
       allelic variant of -13910C/T polymorphism

<400>  1
tgcagggctc aaagaacaat ctaactggcc tcaaaggaac tc                          42


<210>  2
<211>  25
<212>  DNA
<213>  Artificial sequence

<220>
<223>  Forward Outer Primer (FOP or F3 primer) for LAMP of T allelic
       variant of -13910C/T polymorphism

<400>  2
ggcaatacag ataagataat gtagt                                             25


<210>  3
<211>  38
<212>  DNA
<213>  Artificial sequence

<220>
<223>  unlabeled BIP primer for LAMP of T allelic variant of -13910C/T
       polymorphism

<400>  3
tccacgagga taggtcagtg gaagatggga cgcttgaa                               38


<210>  4
<211>  20
<212>  DNA
<213>  Artificial sequence

<220>
<223>  BOP or B3 for LAMP of T allelic variant of -13910C/T polymorphism

<400>  4
taaaactagg aaaacgcagg                                                   20

<210>  5
<211>  19
<212>  DNA
<213>  Artificial sequence

<220>
<223>  biotin labelled primer (LB) or LAMP of T allelic variant of
       -13910C/T polymorphism

<400>  5
ggggagtagt acgaaaggg                                                    19


<210>  6
<211>  45
<212>  DNA
<213>  Artificial sequence

<220>
<223>  unlabeled FIP primer sequence for LAMP of T allelic variant of
       -22018G/A polymorphism

<400>  6
caggctggtc tcgaactcct gacctcagtg gctcacacct ttgtc                       45


<210>  7
<211>  28
<212>  DNA
<213>  Artificial sequence

<220>
<223>  FOP or F3 primer for LAMP of T allelic variant of -22018G/A
       polymorphism

<400>  7
ccttaaaaac agcattctca gctgggca                                          28


<210>  8
<211>  41
<212>  DNA
<213>  Artificial sequence

<220>
<223>  unlabeled BIP for LAMP of T allelic variant of -22018G/A
       polymorphism

<400>  8
ccaacatggc gaaaacccat tctgggacca caagcacccg c                           41


<210>  9
<211>  20
<212>  DNA
<213>  Artificial sequence

<220>
<223>  BOP or B3 for LAMP of T allelic variant of -22018G/A polymorphism

<400>  9

cctgggctca gtgatcctcc                                                    20

<210>  10
<211>  20
<212>  DNA
<213>  Artificial sequence

<220>
<223>  biotin labelled primer (LB) for LAMP of T allelic variant of
       -22018G/A polymorphism

<400>  10
taaaaataca aaaattagcc                                                    20

<210>  11
<211>  25
<212>  DNA
<213>  Artificial sequence

<220>
<223>  FOP or F3 primer for LAMP of C allelic variant of -13910C/T
       polymorphism

<400>  11
ggcaatacag ataagataat gtatc                                              25

## Description

### Field of the invention

The present invention relates to a method for the detection of lactose intolerance markers in a biological sample by LFA. The present invention relates to related tools and assays useful in said methods.

### Background of the invention

Adult-type Hypolactasia also known as lactase non-persistence or lactose intolerance is a widespread autosomal recessive condition resulting from the physiological decline in activity of lactase-phlorizin hydrolase (LPH) in intestinal cells (Enattah et al., 2002, Nature Genetics, 30, 233-237*;* Misselwitz et al., 2019, Gut, 68:2080-2091).

Lactose intolerance refers to the incapacity to digest lactose due to the deficiency in activity of this dedicated enzyme, located in small intestine. Therefore, undigested lactose molecules are processed by bacteria present in colon flora and generate side products that results in clinical symptoms such as abdominal pain, bloating, flatulence, diarrhea and gas bloat. When intolerance is diagnosed, lactose containing food are excluded from diet potentially increasing risk for nutritional defects, for instance, alteration of bone mineral peak bone mass and predisposition to severe osteoporosis (Mattar et al., 2012, Clinical and Experimental Gastroenterology, 5 113-121*; Misselwitz et al., 2019, supra*).

This condition evaluated to affect roughly 68% (95% CI 64-72) of world population, that can lead to lactose intolerance (LI) *(*Storhaug et al., 2017, Lancet Gastroenterol Hepatol, 2: 738-46). The prevalence is highly variable and is associated to geographic area ranging from 5% in the northwest Europe (Denmark) to almost 100% in some Asian and African populations *(*Di Stefano et al., 2009, Digestive and Liver Disease, 41, 474-479*;* Harrington et al., 2008, Int J Clin Pract, October, 62, 10, 1541-1546*; Storhaug et al., 2017, supra).* The Global Lactose Intolerance Treatment Market (including food supplements, foodstuffs, enzymes and treatment/diagnostic) is expected to register a CAGR of 7.10% and reach USD 41 Billion by 2025.

The current standard method is breath hydrogen test after ingestion of 50 g lactose where the fermentation of undigested lactose by the intestinal flora is monitored by measuring hydrogen exhalation at time intervals (Enko et al., 2014, Gastroenterology Research and Practice, 464382*;* Marton et al., 2012, Aliment Pharmacol Ther, 35: 429-440*).* Despite being widely used, the test relies on the activity of bacterial flora that can be altered by antibiotics or acidic colonic pH leading to false-negative results. Furthermore, the test could be very painful for patients due to the generation of high amount of hydrogen, carbon dioxide and methane. Diagnostic performances compared to the genetic polymorphism C/T-13910 have shown good correlation with an overall sensitivity of 88% and specificity of 85% (*Marton et al., 2012, supra).*

Measurement of total and reducing sugars in stool as well as fecal pH are indirect tests for lactose malabsorption in children. The stool pH in infants with lactose intolerance is typically below 5.5 to 6.0. Up to 0.25% of total/reducing sugars is considered normal. In young breastfed infants with physiological lactose malabsorption, concentrations may be higher. The test is not recommended in children older than 2 years of age due to a high rate of false-negative results (Heine et al., 2017, World Allergy Organization Journal, 10:41).

Other well-established assay, although being very invasive, relies on direct measurement of lactase activity performed on biopsy sample of patients' small intestine. Quick lactase test (Biohit) with a cutoff value of 10U/g protein can effectively identify patients with severe duodenal hypolactasia with sensitivity and specificity of 95% and 100%, respectively compared to C/T-13910 polymorphism (Kuokkanen et al., 2006, Endoscopy, 38 (7): 708-712).

Genetically, the expression of the lactase encoding gene (LCT) is influenced by a regulatory region located in the MCM6 gene, approximately 14 kb upstream LCT. Several variants in nucleotide, especially single nucleotide polymorphism (SNP), have been associated with Lactase persistence and non-persistence. SNP are the simplest form of DNA sequence variability (only one modified nucleotide).

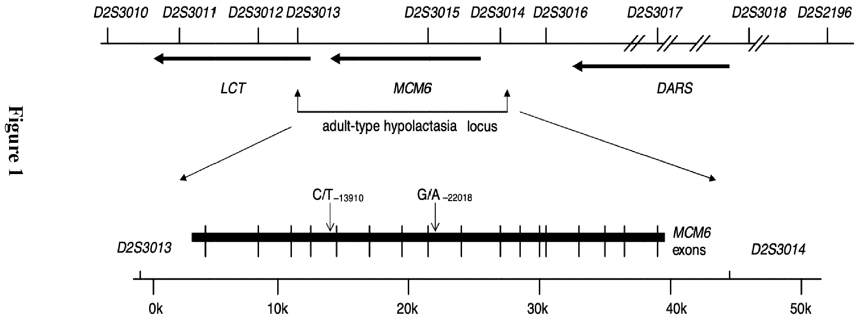

In Europe, the main variant was identified as the -13910C > T. Other variant such as -13907C > G and -13915T > G are found in immigrants from Africa and Middle East (Abildgaard et al., 2018, Clinica Chimica Acta, 482, 50-56). In practice, individuals harboring on or both copies of the T allele (C/T or T/T) at this locus (-13910C > T) have enough lactase activity. Conversely, individuals with no copy of the T allele (C/C) are unable to digest lactose and are classified as lactose intolerant (Carlos et al., 2017, Biotechnology Reports, 16, 21-25; *Enattah et a*/*., 2002, supra* as represented on **Figure 1**). Several groups have investigated the correlation between genetic test and lactase activity by breath test, finding good agreement between the two methods (*Di Stefano et al., 2009, supra; Marton et al., 2011, supra).* A positive genetic test indicates if the patient has genetic predisposition of a lactase non-persistence but do not give information on actual patient symptoms (*Di Stefano et al., 2009, supra*).

Currently, genetic tests are performed in well-equipped laboratory, free of contaminants, in order to perform high fidelity PCR amplification of DNA. Isothermal amplification technics are new DNA amplification assays. Loop-Mediated Isothermal Amplification (LAMP) is a method developed in 2000 by *Notomi et al.* (Notomi et al., 2000, Nucleic Acids Res., 28(12):E63*)* for the amplification of DNA at 50-70°C. This method employs a DNA polymerase and a set of four specially designed primers that recognize a total of six distinct sequences on the target DNA. An inner primer containing sequences of the sense and antisense strands of the target DNA initiates LAMP. The following strand displacement DNA synthesis primed by an outer primer releases a single-stranded DNA. This serves as template for DNA synthesis primed by the second inner and outer primers that hybridize to the other end of the target, which produces a stem-loop DNA structure. In subsequent LAMP cycling one inner primer hybridizes to the loop on the product and initiates displacement DNA synthesis, yielding the original stem-loop DNA and a new stem-loop DNA with a stem twice as long. The cycling reaction continues with accumulation of 109 copies of target in less than an hour. The final products are stem-loop DNAs with several inverted repeats of the target and cauliflower-like structures with multiple loops formed by annealing between alternately inverted repeats of the target in the same strand. LAMP method has been widely used for bacteria, parasitic and viral infections; antibiotic resistance and bacterial toxins (Schoepp et al., 2017, Sci. Transl. Med. 9, eaal3693*;* Modak et al., 2016, Infectious Diseases: Research and Treatment, 9, 1-9*;* Yin et al., 2016, Letters in Applied Microbiology, 63, 16-24*;* Li et al., 2019, Infection and Drug Resistance, 12, 2343-2353*;* Wang et al., 2017, Front. Microbiol., 8:192*)*. Recently, *Carlos et al., 2017, supra* have used LAMP for the characterization of Single nucleotide polymorphism associated to lactose intolerance using buccal cell DNA samples. *Abildgaard et al., 2018, supra* used a commercially LAMP kit for lactose intolerance and a detection using melting curve analysis in blood. These tests are unfortunately not considered as Point-of-Care and require highly skilled staff.

Consequently, there is a very high need to develop accurate cost-efficient methods and tools for an easy detection of lactose intolerance that could be carried out at the point of care testing.

### Summary of the invention

The present invention relates to the finding of a very accurate method for the identification of lactose intolerance based on new generation of Lateral flow assay (LFA) combined with an isothermal DNA amplification (LAMP) which is quicker and easier-to-perform than the classical gold standard genetic analyses which makes it suitable for home testing. In particular, the method of the invention provides an unexpectedly advantageous rapid assay combining a (i) quick DNA isolation using an extraction buffer of the invention, (ii) LAMP amplification of the isolated DNA using primers of the invention and (iii) detection of amplified DNA with a lateral flow immunoassay. The detection of lactose intolerance is based on the detection of the presence or absence of a specific genetic polymorphism C/T-13910 and/or G/A-22018 variants and especially the presence or absence of the T allele or A allele (specific for a lactase persistence) from a biological sample obtained from a patient. The method of the invention provides an indirect information regarding lactose intolerance: if one or both alleles are present, it is indicative of lactase persistence and therefore of lactose tolerance. In case of absence of T or A alleles, it is indicative of lactase deficiency (unable to produce lactase, the enzyme responsible of the digestion of lactose) and therefore of lactose intolerance.

An object of this invention is to provide a non-invasive (e.g. based on sampling of saliva with a swab or finger prick blood sample) method for sensitive and specific detection of lactose intolerance of a subject.

It is advantageous to provide a method for sensitive (e.g. >90%) and specific (e.g. >90%) detection of a lactase persistence biomarker in sample such as a blood or saliva sample.

It is advantageous to provide a method for simple, sensitive and specific detection of the presence or absence of at least one lactase persistence biomarker, in particular genetic polymorphism C/T-13910 variants and/or G/A-22018, wherein said method detects the presence a copy of the T allele (-13910 C/T) or the presence of the A allele (-22018 G/A).

It is advantageous to provide a method for simple, sensitive and specific detection of the presence or absence of a lactase persistence biomarker within less than 1 hour.

It is further advantageous to provide a DNA extraction buffer which allows to extract DNA from a biological sample, in particular from a blood or saliva sample in an efficient manner.

It is advantageous to provide primers which allow efficient DNA amplification specifically for T allele (-13910 C/T) or A allele (-22018 G/A) by LAMP which are specific for a lactase persistence.

Another object of this invention is to provide a test kit for sensitive and specific detection of the presence or absence of T allele (-13910 C/T) or A allele (-22018 G/A) in a sample.

Another object of this invention is to provide a DNA extraction buffer which allows to extract DNA from a biological sample, in particular from a blood or saliva sample in an efficient manner.

Another object of this invention is to provide sets of primers which allow DNA amplification specifically for T allele (-13910 C/T) or A allele (-22018 G/A) by LAMP in an efficient manner.

Objects of this invention have been achieved by providing a lateral flow immunoassay according to claim 1, a method according to claim 10 or a kit according to claims 15 or 16.

Disclosed herein, according to a first aspect of the invention, is a lateral flow immunoassay device for qualitative detection of the presence of a T allele (-13910 C/T) or A allele (-22018 G/A) in a biological sample, said lateral flow immunoassay device comprising a backing support and, on said backing support, a capillary flow array and a wicking pad. The capillary flow array comprises **i**) a sample receiving pad located at one end of the backing support; **ii**) a conjugate release pad being distinct from the sample receiving pad or included to the sample receiving pad and being in capillary contact with the sample receiving pad and being impregnated with at least one detection reagent comprising a first binder specific and binding to at least one labelled allele selected from T allele (-13910 C/T) or A allele (-22018 G/A), said first binder being conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising at least one control reagent line, said capture reagent and control reagent arrays being immobilized on the detection membrane, wherein the wicking pad is located at the other end of the backing support and being in capillary contact with the detection pad and wherein said at least one reagent test line comprises an antibody against the label of said labelled amplified allele and said at least one control reagent line comprises a control reagent having a specific affinity for the first binder of the detection reagent. The said at least one detection reagent are diffusively releasable from the conjugate release pad. The conjugate release pad comprising a detection reagent support wherein the said at least said one detection reagent is bound in a capillary releasable manner, receives the biological sample (e.g. amplified DNA from blood or saliva) from the sample receiving pad and releases the detection reagent from its detection reagent support and a first immuno-complex is formed when a T allele (-13910 C/T) or an A allele (-22018 G/A) analyte is present in the sample and is combined with the detection reagent. The first immuno-complex migrates to the detection pad where it binds to the said at least one reagent test line comprising the said at least one antibody against the label for the said allele. The excess of detection reagent is captured by the said control reagent line.

Disclosed herein, according to another aspect of the invention, is a method for detecting lactose tolerance markers in a biological sample, said method comprising detecting the presence (or absence) of T allele (-13910 C/T) and/or of A allele (-22018 G/A) in a biological sample by LFA.

According to another aspect of the invention, is provided a kit for detection of lactose intolerance, said kit comprising at least one lateral flow immunoassay device according to the invention.

Disclosed herein, according to a first aspect of the invention, is provided a DNA extraction buffer.

Disclosed herein, according to a first aspect of the invention, is provided a set of LAMP primers for the specific amplification of at least one lactase persistence biomarker selected from T allele (-13910 C/T) and/or A allele (-22018 G/A).

### Description of the figures

**Figure 1** is a schematic representation of the physical map of adult lactase non-persistence locus with regulatory regions as described in *Enattah et al. 2002, supra.*

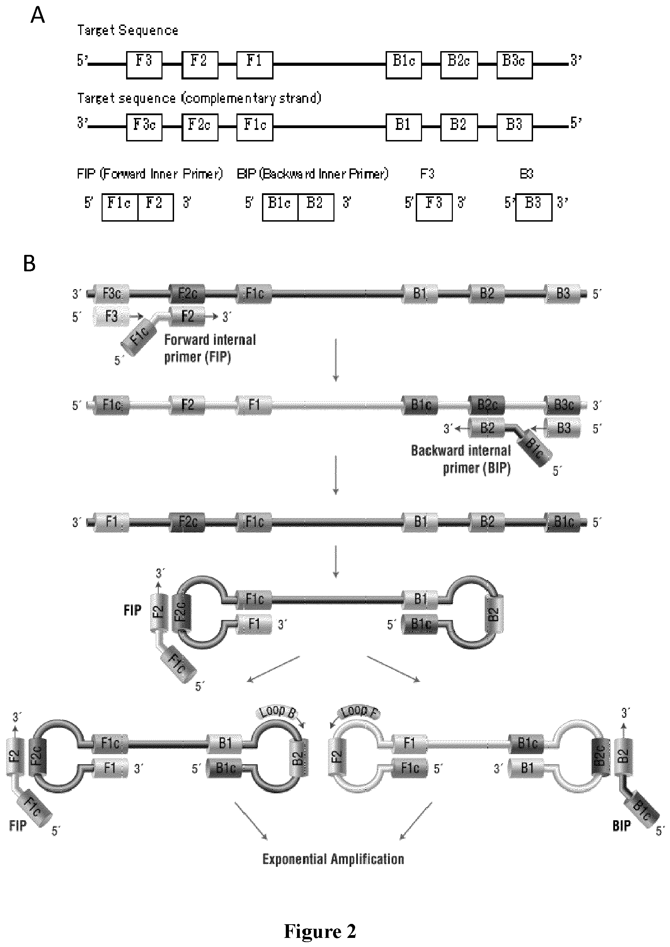

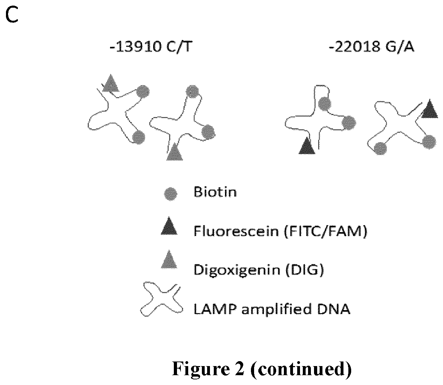

**Figure 2** is a schematic representation of the design of LAMP primers (5'-3') and steps **(A)** Representation of designed primers for LAMP (primer design) *ref:* *https:*//*primerexplorer.jp*/*e*/*v5_manual*/*index.html, primer design;* **(B)** Loop-mediated isothermal amplification (LAMP) which uses 4-6 primers recognizing 6-8 distinct regions of target DNA. A strand-displacing DNA polymerase initiates synthesis, and two of the primers form loop structures to facilitate subsequent rounds of amplification are used as described in Alhassan et al., 2015, Trends in Parasitology, 31(8*)* and the obtained amplified DNA products (amplicons), wherein amplified T-alleles (-13910C/T) are labelled with DIG and biotin and amplified A-alleles (-22018G/A) will be labelled with FAM or FITC and biotin **(C).**

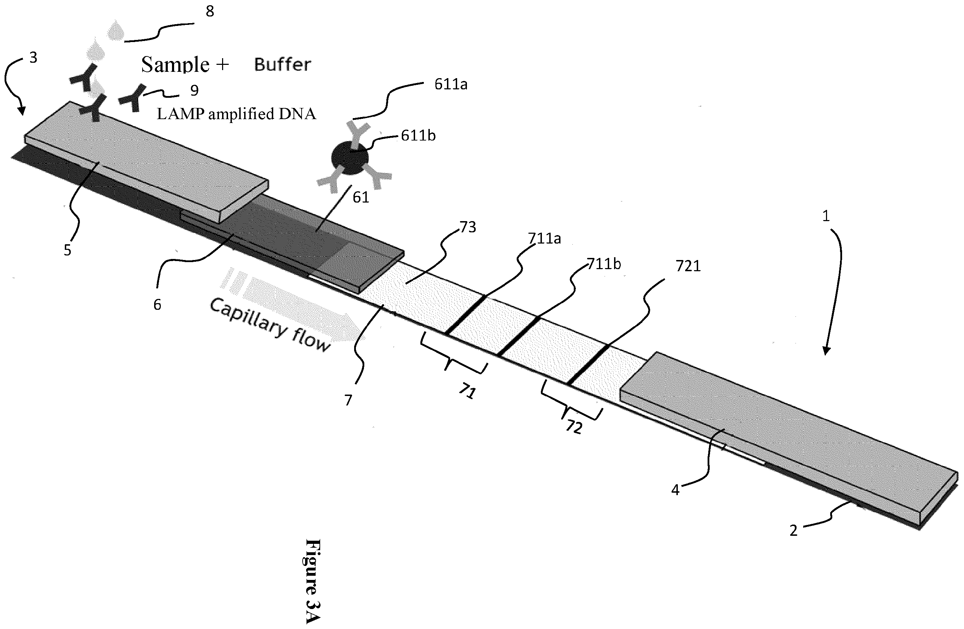

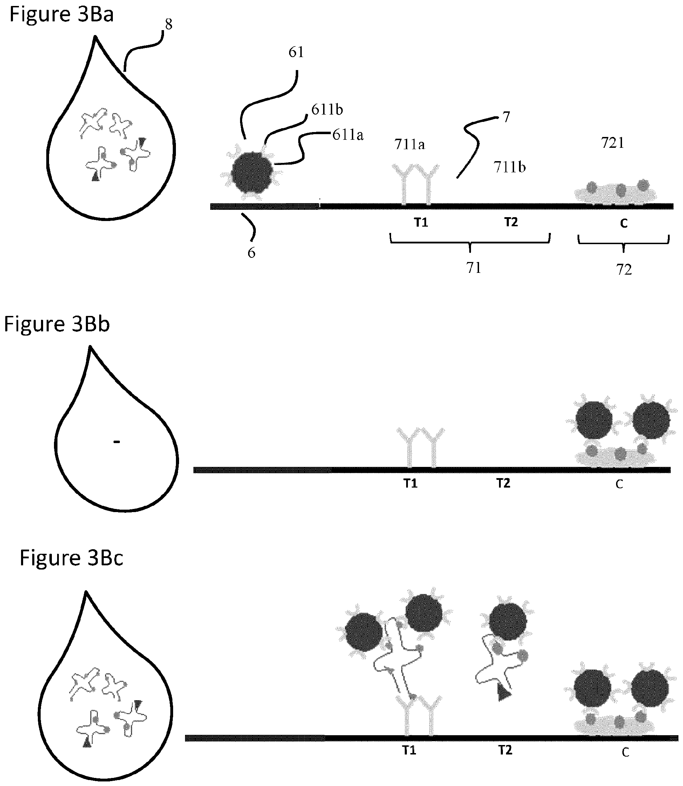

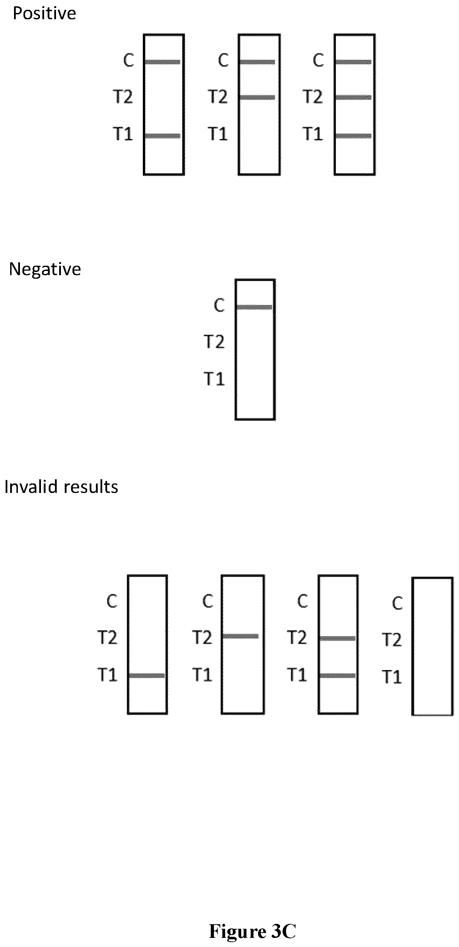

**Figure 3** is a schematic representation of a lateral flow immunoassay of the invention (**A**), the main steps used in the LFA detection step (**B**) of the invention and its read outs (**C**). **B:** In a method of the invention, in particular using a lateral flow immunoassay of the invention such as described under **A** for the detection of the labelled amplicons, the sample (8) containing the analyte (labelled amplified DNA allele marker(s) (9)) is subjected to the sample receiving pad and migrates to the conjugate release pad comprising detection reagents for said labelled amplified allele markers (first binder with first label moiety) which are then released and migrate with the sample to the detection pad comprising a detection membrane where at least one antibody against the label for the amplified DNA allele marker is bound (T1: test line 1) and optionally at least one further antibody against another label for the another amplified DNA allele marker (T2: test line 2) and a control agent specific to first binder (C: control line). In case no analyte is present (test negative), the detection reagents will only bind the control line (**Figure 3Bb**) and in case of the analyte is present (test positive), the analyte (amplified labelled DNA allele marker(s)) will bind to the first binder on the surface of the detection pad and detection reagent (e.g. first binder (e.g. streptavidin) with first label moiety) will be attached to the analytes (antibodies fixed to test lines) on the detection pad and the control line (e.g. BSA-Biotin) captures excess of detection reagent **(****Figure 3Bc****); C:** The read outs are in the form of a test line **(T)** formed by the immune-complex formed by the analyte (e.g. amplified labelled DNA allele marker(s)), the capture test reagent(s) (e.g. antibodies to the label of said labelled DNA allele marker(s)) and the detection reagent(s) (e.g. Gold nanoparticle conjugated with streptavidin) and the control line (**C**) is formed by the immune-complex formed by the capture control reagent (e.g. BSA-Biotin complex) and the detection reagent(s) (e.g. Gold nanoparticle conjugated with streptavidin). Several examples of read-out are provided as an example.

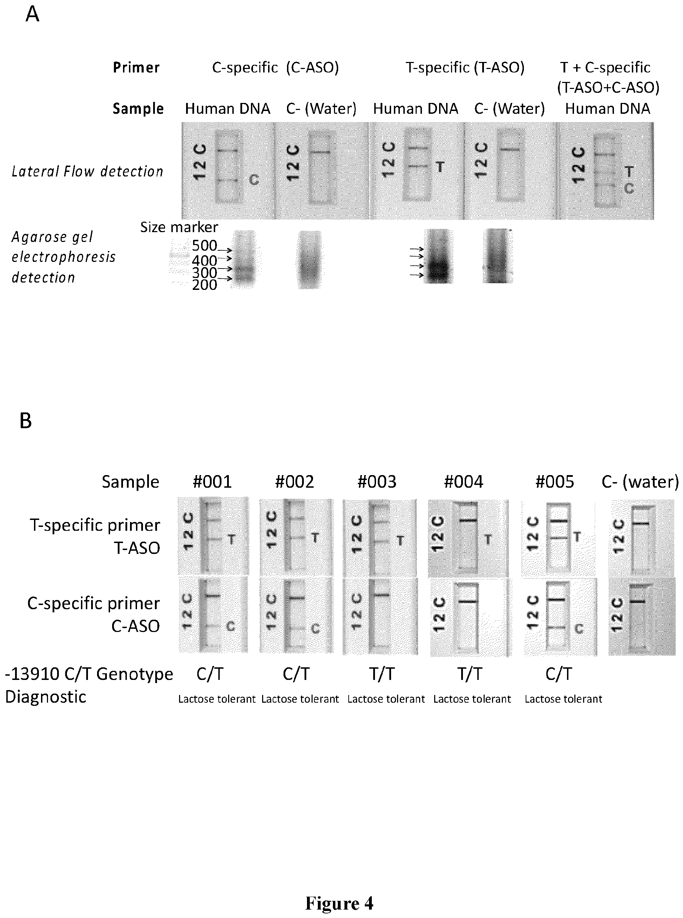

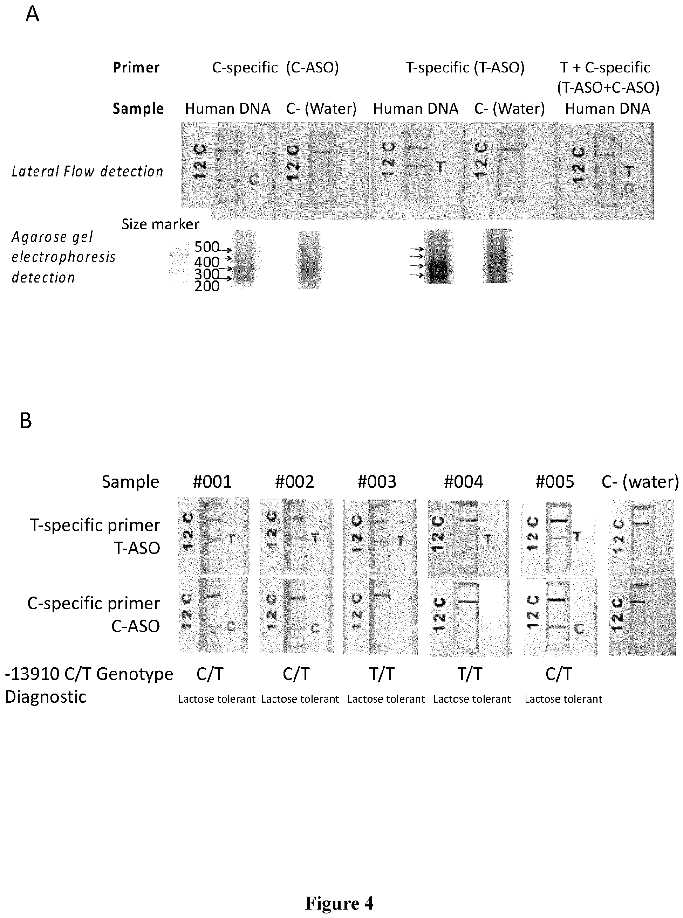

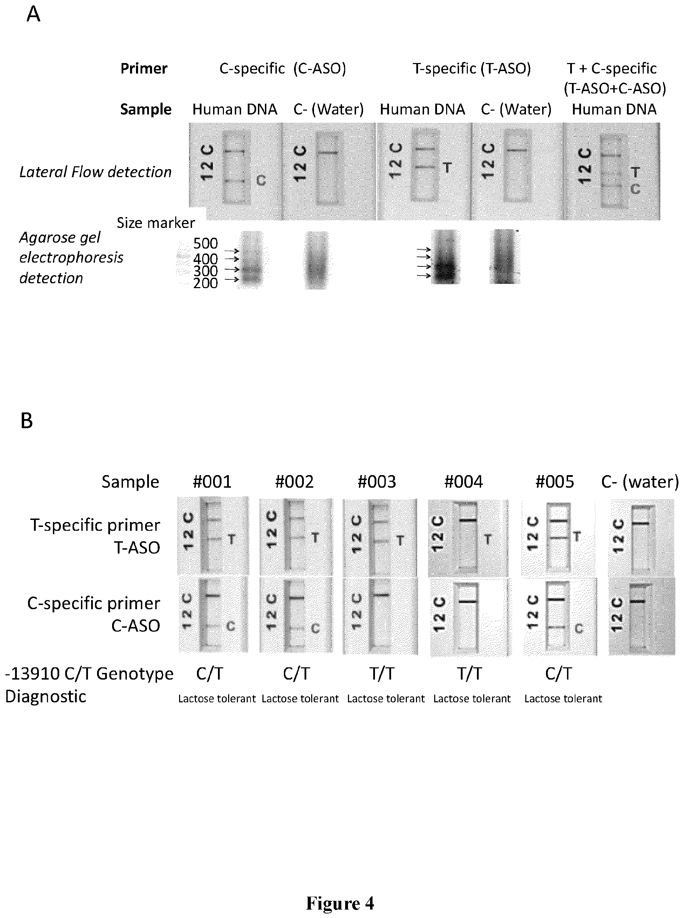

**Figure 4** shows the results of mixed DNA amplification with T-ASO and C-ASO, **(A)** followed by detection with either LFA (top) or gel electrophoresis (bottom or of individual DNA amplifications **(B)** with T-ASO (a) and C-ASO (b), followed by genotype detection with LFA as detailed in Example 1.

### Detailed description

The term "biological sample" comprises a saliva sample such as a buccal swab sample and a whole blood sample

The term "whole blood sample" is intended to mean any blood sample diluted or not and/or placed in aqueous solution. Whole blood sample can be a whole blood finger prick.

The term "aqueous solution" is intended to mean containing water or placed in water e.g. purified, distilled, sterile water or water for injection.

The expression "container" refers to any recipient suitable for sample collection, sample dilution and sample preparation such as vials, centrifuge tubes, flasks, Eppendorf tubes, micro-centrifuge tubes, U-shaped tubes, blood collection tubes, thistle tubes, hybridization tubes, capillary tubes, wintrobe tubes, culture tubes, micro-titer tubes, hematocrit tubes and micro-hematocrit tubes.

The expression "DNA extraction buffer" refers to a buffer suitable for the isolation of DNA material from a biological sample. Reference or example of suitable DNA extraction buffers can be found *under* Walker et al., 2019, Biosensors, 9, 117; doi:10.3390/bios9040117*;* Erlichster et al., 2018, The Journal of Molecular Diagnostics, 20(3*);* Soejima et al., 2011, The Journal of Molecular Diagnostics, 13(3*);* Komatsu et al., 2013, J. Infect. Chemother., DOI 10.1007/s10156-013-0630-9*;* Yamamoto et al., 2015, PLOS ONE, DOI:10.1371/journal.pone.0133204*.*

The expression "DNA amplification buffer" refers to a buffer suitable for the amplification for isolated DNA material, in particular by LAMP. Reference or example of suitable DNA amplification buffers can be found under Abbasi et al., 2016, Acta Trop., 162: 20-26, doi: 10.1016/j.actatropica.2016.06.009*;* Noden et al., 2018, PLoS One., 13(2): e0192331*.;* Becherer et al., 2020, Anal. Methods, 12, 717.

The expression "a portable heater" refers to portable heating system that is suitable for heating a LAMP reaction medium such as for example described in Curtis et al., 2012, PLoS One, 7(2): e31432.; Singleton et al., 2014, PLoS One, 9(11): e113693. Or any other electric dry block heater for laboratory tubes such as supplied by ThermoFisher Scientific (Fisher Scientific AG, Neuhofstrasse 11 - CH 4153 Reinach - Suisse).

### Assay of the invention

Referring to the figures, in particular first to Figure 3A, a lateral flow immunoassay device for qualitative detection of the presence of a T allele (-13910 C/T) or A allele (-22018 G/A) in a biological sample, said device comprising:
- a backing support 2
- a capillary flow array 3 on said backing support,
- a wicking pad 4,
the capillary flow array 3 comprises **i)** a sample receiving pad 5 located at one end of the backing support; **ii)** a conjugate release pad 6 being distinct from the sample receiving pad or being integrated to the sample receiving pad and being in capillary contact with the sample receiving pad 5 and being impregnated with at least one detection reagent 61 comprising a first binder specific and binding to at least one labelled allele selected from T allele (-13910 C/T) or A allele (-22018 G/A), 611a conjugated to a first label moiety 611b; **iii)** a detection pad 7 being distinct from the conjugate release pad and being in capillary contact with the conjugate release pad and comprising a detection membrane 73, a capture test reagent array 71 comprising at least one reagent test line 711a and a capture control reagent array 72 comprising one control reagent line 721, said capture reagent and control reagent arrays being immobilized on the detection membrane 73, the wicking pad 4 being at the other end of the backing support 2 and being in capillary contact with the detection pad 7, wherein said at least one reagent test line 711a comprises an antibody against the label of said labelled allele and said control reagent line comprises a control reagent having a specific affinity for the first binder 61. Optionally, the capture test reagent array 71 may comprise further reagent test lines 711b,...n, such as another antibody against another label of a further labelled allele.

The conjugate release pad 6 comprises a detection reagent support 62 wherein the said at least said one detection reagent 61 is bound in a capillary releasable manner, receives sample 8 (e.g. whole blood or saliva) from the sample receiving pad 5 and releases the detection reagent 61 from its detection reagent support 62 and a first immuno-complex is formed when the allele analyte 9 (e.g. T allele (-13910 C/T) or A allele (-22018 G/A)) is present in the sample and is combined with the detection reagent 61. The first immuno-complex migrates to the detection pad 7 where it binds to the said at least one reagent test line 711a or 711b, 711n (depending of the allele present in the sample) from the capture reagent array 71 comprising the antibody against the label of the allele analyte. The excess of detection reagent 61 is captured by the said one control reagent line 721a from the capture control reagent array 72.

According to a particular embodiment, the backing support is made of a non-porous material such as polyvinyl chloride (PVC).

According to another particular embodiment, the sample receiving pad has pores so as to receive the biological sample. Typically, the sample receiving pad comprises an absorbant pad on to which the sample is applied and is typically composed of a woven mesh or cellulose filter. Irrespective of which material is chosen, the sample receiving pad should exhibit consistent absorbency, thickness and density so that uniform wicking rates ensure assay reproducibility. The sample receiving pad should also have low protein binding to avoid loss of analyte. Pre-treatment such as blocking with protein (e.g. BSA, milk protein, gelatin, ...) or polymer/surfactant could be applied to the sample receiving pad to reduce non-specific binding of the analyte. The sample receiving pad could be designed in such way to have red blood cells retention properties.

According to another particular embodiment, the conjugate pad could be made of the same material/support than the sample receiving pad.

According to another particular embodiment, the detection reagent support of the conjugate release pad is typically composed of non-woven glass fiber into which the detection reagent has dried. Once the sample receiving pad has been saturated, the sample flows in to the conjugate release pad where it releases the detection reagent(s) which in turn leave(s) the conjugate release pad and moves with the sample in the detection reagent support to the detection membrane of the detection pad.

According to another particular embodiment, the detection reagent is specific to the allele marker (e.g. biotin), such as streptavidin (first binder specific and binding to the allele analyte) conjugated to colored or fluorescent particles (label moiety).

According to a particular aspect, the label moieties can be gold nanoparticles, latex beads, cellulose beads, fluorescent labels/particles and other colloidal metals and magnetic particles which produce a colored/fluorescent read out or any other particles generally used in lateral flow assays. According to a further embodiment, the label moiety is a gold, cellulose or latex particle.

According to a particular aspect, the detection membrane can be made a membrane of nitrocellulose membrane or cellulose acetate, polyvinylidene fluoride (PVDF), charge-modified nylon, polyethersulfone (PES) or treated/non-treated cellulose. Typically, nitrocellulose membranes exhibit a range of pore sizes (0.05 to 12 µm) and are suitable for the present assay.

According to a particular aspect, capillary flow values expressed in Capillary speed down web for purified water (s/40 mm) between 90 and 180 are suitable for the present assay.

Capture reagents are immobilized across the detection membrane, typically in two capture reagent arrays (test and control reagents), respectively and each of the capture reagent arrays comprise one or more lines. The test line(s) is used to bind the sample target analyte (e.g. antibodies, while the control line consists of agents specific for the detection of the first binders such as species-specific antibodies (e.g. anti-mouse/anti-rabbit antibodies) and is used to demonstrate that the lateral flow immunoassay is performing as it should be.

According to a particular aspect, the wicking pad is composed of a cellulose/cotton fiber.

According to a particular aspect, the lateral flow immunoassay may be inserted into a rigid housing (e.g. a plastic cassette) protecting the capillary flow array and wicking pad and having an opening over the sample receiving pad for loading the sample on to said sample receiving pad and one opening over the capture reagent array for the visual interpretation of the test (presence/absence of colored lines).

According to a particular embodiment, the detection pad may comprise more than one reagent test line in the capture test reagent array. In particular, the capture test reagent array may comprise at least an anti-DIG antibody. In another embodiment, the capture test reagent array may comprise at least an anti-FAM and/or an anti-FITC antibody.

In another embodiment, the capture test reagent array comprises at least one anti-DIG antibody and at least one anti-FAM and/or an anti-FITC antibody.

According to a particular embodiment, the control agent specific to first binder is BSA-biotin.

### Preparation of an immunoassay of the invention

A sample receiving pad can be purchased from Ahlstrom-Munksjö^{®} as a single layer plasma separation media consisting of high purity natural or synthetic fibers for amplified DNA diagnosis such as Ref. A1660, A8950 or A8951. Pre-treated or not conjugate pads can be made of glass and/or polyester fibers such as Ahlstrom-Munksjö Ref. A8964 or A8914. According to a particular embodiment, the detection membrane can be made of nitrocellulose such as Hi-Flow^{™} Plus from Millipore, HF-120, HF-135 or HF-180, representing the capillary flow time (sec/4 cm). Treatment of the detection membrane with blocking proteins such as BSA, casein, gelatin, milk protein or polymers/surfactant could be realized to reduce background signal and to reduce non-specific binding of analyte. The wicking pad serves as absorbent of liquid excess and needs to have good absorbent properties. For example, cotton fiber from Ahlstrom-Munksjö Ref. A222 or A 237 can be used.

Capture reagent for the test lines can be used to a concentration between 0.1 and 2.0 mg/ml in as PBS or other low salt buffer. Addition of blocking/carrier proteins, surfactant or alcohol (ethanol) could be used to optimize the binding and drying of test lines onto the detection membrane. BSA-biotin conjugate with minimal cross-reaction to serum protein and other species, are diluted in PBS or other low salt buffer to a concentration between 0.5 and 2.0 mg/ml can be used as control agent.

All paper and membrane (sample receiving pad, conjugate pad, detection membrane and wicking pad) could be pre-treated and are cut with respective width, depending of the length of the capillary flow array and the superposition of the different membranes. If pre-treatments with blocking solution occur, treated membrane need to be dried in specific condition (such as 37°C, 10% RH (relative humidity), 2 hours or overnight at room temperature and 10% RH). Spraying of the detection reagent on the detection reagent support from the conjugate release pad can be realized with a dispensing machine (BioDot or Kinematic Automation) with air pressure (e.g. 2 PSI), pump dispense flow of e.g. 1 µl/cm and bed speed of e.g. 5 cm/second. Sprayed detection reagent support can be dried with same condition as above. Antibodies (test lines from capture test reagent array) and capture control biotinylated protein (such as BSA-biotin) (control line) are diluted and applied in different position on the detection membrane using similar dispensing machine, (e.g. with pump dispense flow of 1 µl/cm and bed speed of 5 cm/second). Detection membrane is dried under same condition cited above. Assembling of all components of the assay is made by hand or with an automatized process. Assembled cards with different paper and membrane could be covered with a cover tape (transparent adhesive plastic) in order to protect the assay integrity. Assembled cards are then cut in strip suitable for the assay (e.g. with a width between 3.0 and 7.0 mm). Cut strips can then be assembled in a plastic cassette in order to protect the strip, pouched with a desiccant in an aluminum pouch and sealed with a thermo-sealing machine. All production and assembling steps take place in clean room with controlled temperature (<25°C) and humidity (<40% RH). Assembling of the final kit with accessories (optional), test in sealed pouch, instruction for use in a kit box can be done in a non-controlled environment.

### Methods of the invention

According to one particular aspect of the invention, is provided a method for detecting the presence of T allele (-13910 C/T) and/or of A allele (-22018 G/A) in a sample, said method comprising:
a) Providing a sample comprising DNA material (amplicons) in aqueous solution, wherein in said DNA material has been subjected to specific amplification and labelling targeting DNA from T allele (-13910 C/T) and/or of A allele (-22018 G/A);
b) Subjecting the said sample to a lateral flow immunoassay comprising i) a conjugate release pad comprising detection reagents for said labelled amplified alleles and ii) a detection pad comprising a detection membrane where at least one antibody against the label of said amplified allele(s) is bound;
c) Detecting the presence or absence of a detection line on the detection membrane at the location where the said at least one antibody against the label of said amplified allele(s) is bound, the presence of said detection line being indicative of T allele (-13910 C/T) and/or of A allele (-22018 G/A) in said sample.

According to another particular aspect of the invention, is provided a method for detecting lactose tolerance markers in a sample, said method comprising:
i. Providing a sample comprising DNA material (amplicons) in aqueous solution, wherein in said DNA material has been subjected to specific amplification and labelling targeting DNA from T allele (-13910 C/T) and/or of A allele (-22018 G/A);
ii. Subjecting the said sample to a lateral flow immunoassay comprising i) a conjugate release pad comprising detection reagents for said labelled amplified alleles and ii) a detection pad comprising a detection membrane where at least one antibody against the label of said amplified allele(s) is bound;
iii. Detecting the presence or absence of a detection line on the detection membrane at the location where the said at least one antibody against the label of said amplified allele(s) is bound, the absence of said detection line being indicative of the absence of a T allele (-13910 C/T) and/or of A allele (-22018 G/A), those being lactose tolerance markers.

According to a particular aspect, the detection of the presence of a T allele (-13910 C/T) and/or of A allele (-22018 G/A) according to a method of this invention or by an immunoassay of the invention is indicative of lactase persistence also called lactose tolerance.

According to a particular aspect, the detection of the absence of a T allele (-13910 C/T) and/or of A allele (-22018 G/A) according to a method of this invention or by an immunoassay of the invention is indicative of a lactase non persistence or lactose intolerance.

According to a particular aspect, the amplified DNA material (amplicons) is obtained from LAMP amplification.

According to another particular aspect, the amplified DNA material (amplicons) used in a method of the invention has been obtained by LAMP amplification of DNA extracted from a biological sample.

According to a further another particular aspect, the DNA material is extracted from a biological sample before LAMP amplification in an extraction step where a biological sample (e.g. whole blood or saliva) is subjected to a DNA extraction step in presence of a extraction buffer according to the invention, said extraction buffer comprising from about 0.1 to about 0.5 mM EDTA (e.g. 0.2 mM) and from about 20 to about 50 mM NaOH (e.g. 25 mM).

According to another further particular aspect, the DNA extraction step is carried out at about 95-100°C (e.g. about 98°C) for about 2 to 5 minutes.

According to a particular aspect, the amplification could be done also at room temperature using the same extraction buffer (e.g. 5 min at room temperature, 20-25°C).

According to a particular aspect, the DNA extraction buffer according to the invention allows achieving DNA extraction from a biological sample in less than 10 minutes without specific equipment.

According to a particular aspect, the amplified DNA material (amplicons) is obtained from LAMP amplification wherein DNA from T allele (-13910 C/T) is specifically amplified and labelled using a set of the following primers selected from the following group:
- **a FIP primer** comprising a sequence of **SEQ ID NO: 1;**
- **a FOP or F3 primer** comprising a sequence of **SEQ ID NO: 2;**
- **a BIP primer** comprising a sequence of **SEQ ID NO: 3;**
- **a BOP or B3 primer** comprising a sequence of **SEQ ID NO: 4;**
- **a LB primer** for labelling the resulting amplified DNA, wherein the resulting labeled amplified DNA can be detected by the detection reagent of a lateral flow assay; and wherein either the FIP or the BIP primer is labelled with a labelling group for fluorescence detection such as a Fluorescein amidite (FAM) or Fluorescein isothiocyanate (FITC) label or for immune detection such as Digoxigenin (DIG) label.

According to a further particular aspect, the FIP primer is labelled with a labelling group.

According to a further particular aspect, when the FIP primer is labelled with a labelling group, said primer is labelled either at the 5' or 3' end (or said on its F1c part or on the B1c part), in particular at the 5'.

According to another particular aspect, the BIP primer is labelled with a labelling group at the 5' or 3' end of the primer.

According to a further particular aspect, the LB primer used for LAMP amplification of DNA from T allele (-13910 C/T) comprises a sequence of **SEQ ID NO: 5.** This primer allows the labelling of the amplified DNA at the 5' end with biotin in order to be detected with detection particles labelled with streptavidin. LB primer itself can also be labelled either at 3' or 5' end of LB primer.

According to another particular aspect, the amplified DNA material (amplicons) is obtained from LAMP amplification wherein DNA from A allele (-22018G/A) is specifically amplified and labelled using a set of the following primers selected from the following group:
- **a FIP primer** comprising a sequence of **SEQ ID NO: 6;**
- **a FOP or F3 primer** comprising a sequence of **SEQ ID NO: 7;**
- **a BIP primer** comprising a sequence of **SEQ ID NO: 8;**
- **a BOP or B3 primer** comprising a sequence of **SEQ ID NO: 9;**
- **a LB primer** for labelling the resulting amplified DNA, wherein the resulting labeled amplified DNA can be detected by the detection reagent of a lateral flow assay; and wherein either the FIP or the BIP primer is labelled with a labelling group for fluorescence detection such as a Fluorescein amidite (FAM) or Fluorescein isothiocyanate (FITC) label or for immune detection such as Digoxigenin (DIG) label.

According to a further particular aspect, the LB primer used for LAMP amplification of DNA from A allele (-22018G/A) comprises a sequence of **SEQ ID NO: 10.** This primer allows the labelling of the amplified DNA with biotin in order to be detected with detection particles labelled with streptavidin.

According to a particular aspect, the DNA material (amplicons) is obtained from LAMP amplification using at least one set of the FIP, FOP or F3, BIP, BOP or B3 and LB primers as described above.

According to another particular aspect, the DNA material (amplicons) is obtained from LAMP amplification using the two sets of the FIP, FOP or F3, BIP, BOP or B3 and LB primers as described above. In this case, the label of the FIP or the BIP primer of one set is then different from the label of the FIP or the BIP primer of the other set. According to a particular embodiment, each set might be useful depending on the specific ethnicity of the patients to be tested. For example, Caucasian population from Europe are more likely -13910 C/T and the Middle East or African population -22018G/A.

Advantageously, a kit comprising both set of primers would be useful for any ethnicity of a population.

According to a particular aspect, LAMP amplification is carried at amplification temperature from about 70 to about 80°C.

According to a particular aspect, LAMP amplification is carried at amplification temperature for about 20 to about 30 minutes.

According to a particular aspect, provided DNA material (amplicons) in aqueous solution, is DNA material that has been subjected to specific amplification and labelling targeting DNA from T allele (TT or TC allelic variant) from -13910C/T.

According to another further particular aspect, is provided a method according to the invention wherein at least one anti-DIG antibody, for example selected from Jackson Immuno Research Europe or Biotechne AG or a combination of those, is bound to the detection membrane of a lateral flow immunoassay as a reagent test line and the detection of the presence of DIG labelled T allele (-13910 C/T) is carried out under step c).

According to another further particular aspect, is provided a method according to the invention wherein at least anti-FAM or anti-FITC antibody, for example selected from Jackson Immuno Research Europe or Biotechne AG or a combination of those, is bound to the detection membrane of a lateral flow immunoassay as a reagent test line and the detection of the presence of FAM or FITC labelled T allele (-13910 C/T) is carried out under step c).

According to a particular aspect, provided DNA material (amplicons) in aqueous solution, is DNA material has been subjected to specific amplification and labelling targeting DNA from A allele from -22018 G/A.

According to another further particular aspect, is provided a method according to the invention wherein at least one anti-DIG antibody is bound to the detection membrane of a lateral flow immunoassay as a reagent test line and the detection of the presence of DIG labelled A allele (-22018 G/A) is carried out under step c).

According to another further particular aspect, is provided a method according to the invention wherein at least one anti-FAM or anti-FITC antibody is bound to the detection membrane of a lateral flow immunoassay as a reagent test line and the detection of the presence of FAM or FITC A allele (-22018 G/A) is carried out under step c).

According to another further particular aspect, is provided a method according to the invention wherein both the presence (or absence) of T allele (-13910 C/T) and of A allele (-22018 G/A) is detected. In this case, the detection membrane of the lateral flow immunoassay comprises a first reagent test line for detecting a first label on A-allele (e.g. FAM/FITC or DIG) and a second reagent test line for detecting a second label on T-allele (e.g. DIG or FAM/FITC), wherein the first label and the second label are not the same.

According to another further particular aspect, is provided a method for detecting lactose tolerance markers that can be performed in less than 30-45 min compared to classical methods (>1h).

### Kits of the invention

According to one embodiment of the invention, is provided a kit for detection at least one lactase persistence biomarker in a biological sample, said kit comprising at least one lateral flow immunoassay device according to the invention.

According to a particular aspect of the invention, the said at least one lateral flow immunoassay device is sealed in a pouch with a desiccant.

According to a particular aspect of the invention, the said at least one lateral flow immunoassay device is assembled in a plastic cassette.

According to a particular aspect of the invention is provided a kit of the invention may further comprise at least one of the following:
- a sample collection device (e.g. pipette or automatic lancet for finger prick blood sample or capillary tube, capillary pipette, or swab for the sampling of DNA from buccal cells);
- a container with a buffering solution for dilution of the sample or for running the test with pure sample (e.g. Low-density polyethylene (LDPE) plastic bottle with cap and dropper or any other closed plastic bottle with dropper);
- instructions for use.

According to a particular aspect of the invention is provided a kit of the invention further comprising or more of the following:
- a disinfection pad;
- an adhesive plaster;
- a data reader for reading the assay results and optionally transmitting to a database (e.g. patient hospitalization data set).
- a portable heater to heat the LAMP reaction medium at 70-80°C.

According to a particular aspect of the invention is provided a kit of the invention further comprising a lysis/extraction buffer for the DNA extraction from a biological sample.

According to a particular aspect of the invention is provided a kit of the invention further comprising a DNA amplification (LAMP) buffer.

According to another aspect of the invention, is provided a kit for detection of at least one lactase persistence biomarker in a biological sample, said kit further comprising at least one primer according to the invention.

According to a particular aspect of the invention is provided a LAMP kit for amplification and labelling DNA from T allele (-13910 C/T) and/or of A allele (-22018 G/A), said LAMP kit comprising at least one primer of the invention.

According to a particular aspect of the invention is provided a LAMP kit for amplification and labelling DNA from T allele (-13910 C/T) and/or of A allele (-22018 G/A), said LAMP kit comprising a set of the following primers:
- **a FIP primer** comprising a sequence of **SEQ ID NO: 1;**
- **a FOP or F3 primer** comprising a sequence of **SEQ ID NO: 2;**
- **a BIP primer** comprising a sequence of **SEQ ID NO: 3;**
- **a BOP or B3 primer** comprising a sequence of **SEQ ID NO: 4;**
- **a LB primer** for labelling the resulting amplified DNA with biotin, wherein the resulting labeled amplified DNA can be detected/captured by the detection reagent of a lateral flow assay; and wherein either the FIP or the BIP primer is labelled with a labelling group for immune or fluorescence detection such as a Fluorescein amidite (FAM) or Fluorescein isothiocyanate (FITC) label or for immune detection such as Digoxigenin (DIG) label;
   and/or
- **a FIP primer** comprising a sequence of **SEQ ID NO: 6;**
- **a FOP or F3 primer** comprising a sequence of **SEQ ID NO: 7;**
- **a BIP primer** comprising a sequence of **SEQ ID NO: 8;**
- **a BOP or B3 primer** comprising a sequence of SEQ ID NO: **9;**
- **a LB primer** for labelling the resulting amplified DNA with biotin, wherein the resulting labeled amplified DNA can be detected/ Captured by the detection reagent of a lateral flow assay; and wherein either the FIP or the BIP primer is labelled with a labelling group for immune or fluorescence detection such as a Fluorescein amidite (FAM) or Fluorescein isothiocyanate (FITC) label or for immune detection such as Digoxigenin (DIG) label.

Another object of this invention is to provide a DNA extraction kit, said kit comprising an extraction buffer according to the invention.

According to a further aspect, is provided a kit according to the invention for conducting a method of the invention and its use in a method according to the invention.

According to a further aspect, an assay, a method, primers or a kit according to the invention are useful for detection of genetic polymorphism C/T-13910 variants and/or G/A-22018 in a biological sample and therefore the presence or absence of markers of lactose tolerance. According to a further aspect, an assay or a kit according to the invention are useful for detection of lactose intolerance.

According to a particular embodiment, the method, the primers and assay according to the invention allow the detection of a lactose intolerance in a very rapid manner (less than 30 minutes) with a high selectivity and high sensitivity (e.g. >90%). The primers of the invention detect genetically the lactose intolerance even without clear symptoms, compared to other methods which required symptoms. Moreover, this test is not invasive and do not require the ingestion of large quantity of lactose (e.g. for Breath test) leading to bloating and other symptoms.

Examples illustrating the invention will be described hereinafter in a more detailed manner and by reference to the embodiments represented in the Figures.

### EXAMPLES

The following abbreviations refer respectively to the definitions below:
**BSA** (bovine serum albumin); **PBS** (Phosphate-Buffered Saline); **TBS** (Tris-Buffered Saline); **TBST** (Tris-Buffered Saline with Tween).

### Example 1: Primers according to the invention

In order to validate the efficacy of the primers of the invention and their usefulness in a method according to the invention, the amplification of isolated DNA using primers of the invention was assayed as follows where amplifying C-allelic variant of -13910C/T and amplifying T-allelic variant of -13910C/T were used in parallel:

### a) providing a biological sample obtained from a subject

A sample from buccal cells with a swab is collected. The patient must not eat, drink or smoke about 2 hours before the DNA sampling.

### b) DNA isolation with a commercially available method

DNA extraction from buccal swab was obtained from subjects with a commercial method, nameed NucleoSpin Tissue (Macherey-Nagel) method. A standard DNA sample (Promega) was used as positive control.

### c) DNA amplification

The isolated DNA is amplified using LAMP technology (LavaLAMP, Lucigen) (75°C, 1h) with primers specifically designed to amplify T allele (C/T or T/T) or C allele (C/C or C/T)) of -13910C/T polymorphism, in order to fully characterize allelic variants of samples. In particular, primers are labeled with Fluorescein amidite (FAM) or Digoxigenin (DIG) and biotin.

The amplification step employs 5 template-specific primers as described in Table 1 below, of which one is an Allele Specific Oligo (ASO) primer, capable of recognizing only either the - 13910 C allelic variant (C-ASO primer) or the -13910 T allelic form (T-ASO primer). The other 3 primers being in common (FIP, BIP and B3) for both C- and T-allelic variants. The last primer (LB) is biotinylated and is used for the detection of the amplicon on lateral flow assay and is similar for both C- and T-allelic variants.

**Table 1**

| **Primer name** | **DNA sequence (5'-3')** |
|---|---|
| F3 (-13910T) (T-ASO) | GGC AAT ACA GAT AAG ATA ATG TAG T |
| F3 (-13910C) | GGC AAT ACA GAT AAG ATA ATG TAT C |
| (C-ASO) | |
| B3 | TAA AAC TAG GAA AAC GCA GG |
| FIP | TGC AGG GCT CAA AGA ACA ATC TAA CTG GCC TCA AAG GAA CTC |
| BIP | (DIG or FAM)TCC ACG AGG ATA GGT CAG TGG AAG ATG GGA CGC TTG AA |
| LB | (Biotin)GGG GAG TAG TAC GAA AGG G |

The LAMP was carried out using in parallel the two different F3 primers specifically designed to recognize the C-allelic or T-allelic variant of -13910 C/T and the 4 other primers: B3, FIP, BIP and LB for the amplification of both C- and T-allelic variant as described in **Fig. 2A****.**

By running two amplifications for each DNA sample, in parallel, one with the C-ASO primer, the other with the T-ASO, it was therefore possible to discriminate the two allelic variants. The DNA amplification was performed according to the manufacturer protocol (lavaLAMP, Lucigen or Bst3.0 DNA polymerase LAMP (NEB)) as described in Da Silva et al., 2019, Sci Rep. 2019; 9: 4494*;* Yang et al., 2018, Foodborne Pathogens and Disease. Jun 2018.309-331*.* **For detecting the T allelic variant of -13910C/T polymorphism the following LAMP primers were used:**
- **The Forward Inner Primer (FIP):** consists of a F2 region at the 3' end and a F1c region at the 5' end. The FIP sequence used is as follows: tgcagggctcaaagaacaatctaactggcctcaaaggaactc (**SEQ ID NO: 1**) with a FAM label on the F1c part). The F1c part of the FIP primer is designed by taking the 3'-5' complementary DNA sequence and inverting the sequence to have a 5'-3' complementary primer sequence. The F2 part of the primer FIP is designed by selecting the 5'-3' sequence of the gene of interest just after the F3 primer.
- **The Forward Outer Primer (FOP, also called F3 primer):** consists of a region which is complementary to the F3c region of the template sequence. This primer is shorter in length and lower in concentration than FIP. The F3 sequence **(mutant type, T-allele)** used is as follows: ggcaatacagataagataatgtagt **(SEQ ID NO: 2)** (called T-ASO primer) and corresponds to the DNA sequence just before the SNP of interest.
- **The Backward Inner Primer (BIP):** consists of a B2 region at the end of the 3'end and B1c region at the 5'end. The BIP sequence used is as follows:
   tccacgaggataggtcagtggaagatgggacgcttgaa (**SEQ ID NO: 3**). The B1c part of the primer BIP is formed by selecting the 5'-3' sequence of the gene of interest. The B2 part of the BIP primer is formed by taking the 3'-5' complementary DNA sequence and invert the sequence to have a 5'-3' complementary primer sequence. The BIP could also be labelled (B1c part) with the same label used for FIP but only if the FIP is not labelled.
- **The Backward Outer Primer (BOP also called B3).** The BOP sequence used is as follows: taaaactaggaaaacgcagg **(SEQ ID NO: 4)** which corresponds to the 3'-5' complementary DNA sequence at the end of the sequence of interest;
- **The biotin labelled primer (LB).** The LB sequence used is as follows: ggggagtagtacgaaaggg **(SEQ ID NO: 5)** which corresponds to a DNA sequence between the B1c and B2 with a biotin label;

**For detecting the C allelic variant of -13910C/T polymorphism the same LAMP primers were used, only the F3 primer and labelling of FIP or BIP were modified as follow:**
- **The Forward Inner Primer (FIP):** consists of a F2 region at the 3' end and a F1c region at the 5' end. The FIP sequence used is of **SEQ ID NO: 1** with a DIG label on the F1c part.
- **The Backward Inner Primer (BIP):** consists of a B2 region at the end of the 3'end and B1c region at the 5'end. The BIP sequence used is of **SEQ ID NO: 3).** The BIP could also be labelled (B1c part) with the same label used for FIP but only if the FIP is not labelled.
- **The Forward Outer Primer (FOP, also called F3 primer):** consists of a region which is complementary to the F3c region of the template sequence. This primer is shorter in length and lower in concentration than FIP. The F3 sequence **(wild type, C-allele)** used is as follows: ggcaatacagataagataatgtatc **(SEQ ID NO: 11),** (called C-ASO primer) and corresponds to the DNA sequence just before the SNP of interest.

First, non-cyclic steps are carried out to generate stem loop DNA with dumbbell-shaped structure at both ends with the primers defined above and then the obtained DNA structures serve as an initiator for LAM cycling as described in **Figure 2B****.**

In particular, tubes containing 25 µl of LAMP reaction buffer containing specific LAMP saline buffer, MgCl₂ and DNA polymerase enzyme and deoxyribonucleotide triphosphate (dNTPs) with specific designed DNA primers as described above.

Final concentration of the primer in the LAMP reaction is 0.2 µM for F3/B3 and 1.0 µM for BIP/FIP and LB. The LAMP reaction is performed according to the manufacturer protocol (lavaLAMP, Lucigen), summarized below in Table 2:

**Table 2**

| **Components** | **Final concentration** | **Volume for 1 reaction (µl)** |
|---|---|---|
| LAMP Buffer (2x) | 1x | 12.5 |
| Primer Mix (10x) | 1x | 2.5 |
| Water | - | 9 |
| Extracted DNA | - | 1 |

All the LAMP reaction components are added in a sterile 1.5 ml tubes. The LAMP amplification is run during 20-60 minutes at 65-72°C (according to manufacturer). Temperature was optimized during the development of the test. Two lateral flow (LFA) tests were run in parallel, one with C-ASO primers, amplifying C-allelic variant of -13910C/T and one with T-ASO primer, amplifying T-allelic variant of -13910C/T. The C-ASO primer were labelled with DIG and biotin and the T-ASO primer with FAM and biotin to be detected on a LFA test line 1 (anti-DIG antibody) and a LFA test line 2 (anti-FAM antibody) as described below:

### d) Amplified DNA detection

The obtained amplified DNA (amplicons) are diluted and detected by a nucleic acid lateral flow assay (LFA) incorporating antibodies which capture and allow visualization of doublestranded amplification products containing selected binding partners, FAM and biotin, as well as DIG and biotin. The amplicon detection takes only 5-10 minutes, and the result is detectable by eye due to an aggregation of detection reagent particles at the capture lines as detailed below:
A lateral flow immunoassay of the invention was prepared as follows:

### Production of a "Lateralflow assay"

The production of a lateral flow assay has been performed in a clean, controlled room (EU GMP Class D), temperature (<25°C) and humidity (<40% RH). The strips were cut with a width of 5 mm (± 0.5 mm) and stored in aluminum pouches with desiccants at room temperature.

The lateral flow assay is composed of a sample receiving pad (glass fiber material of 20 mm such as such as Ahlstrom-Munksjö Ref. A8964), used also as conjugate release pad, where the detection reagent is located on the top end of the pad (e.g. sprayed with streptavidin labelled gold, cellulose, latex, metallic nanoparticles as first binder to the analyte). This sample/conjugate release pad pasted on backing support and is directly in contact (2 mm) in contact with the detection pad comprising the nitrocellulose membrane (width 25 mm) HF-180 (Merck Millipore) where the different lines (capture test reagent array and capture control reagent array) are located. A wicking pad is located at the end of the nitrocellulose membrane in order to absorb the excess of liquid. Bands of absorbent pad A222 (Ahlstrom-Munksjö) with a width of 21 mm were cut and paste on the upper part of the backing support and used as wicking pad.

### Spotting of test reagents on the detection membrane of the half-sticks

Several test reagents were used as test reagent lines in the capture reagent array:
- The first test line (T1) is composed of anti-DIG antibody specific to Digoxigenin (DIG) that could be from various source such as mouse, rabbit etc (e.g. Abcam ab76907; R&D System MAB7520; Jackson Immuno Research Europe 200-002-156);
- The test line 2 (T2) is an anti-FAM antibody specific to Fluorescein (FAM or FITC) that could be from various source such as mouse, rabbit etc (e.g. Abcam ab 19491; Jackson Immuno Research Europe 200-002-037).

The test reagents were each diluted at a specific concentration, indicated in Table 3 below, in TBS IX. Then, 0.5µL of each diluted test reagent were spotted on the nitrocellulose membrane (detection membrane) and let dry at 25°C, <40% RH for 2 hours to form a test reagent line.

**Table 3**

| **Test reagents** | **Supplier ref.** | **Concentration [mg/ml]** |
|---|---|---|
| Anti-DIG antibody | JIR 200-002-156 | 0.5 |
| Anti-FAM Antibody | JIR 200-002-037 | 0.5 |

A control reagent line of with biotinylated Bovine serum albumin (BSA) protein as control agent was used in the capture control reagent array to detect the detection reagent (streptavidin labelled detection particles).

### Running the LFA assay

3 to 4 drops of the amplified DNA (e.g. about 200 µl) are diluted in a running buffer (Phosphate Buffered Saline solution, with 1-2% tween 20). A blocking agent such as BSA or other protein could be added to the running buffer to reduce non-specific binding.

The diluted amplified DNA is then added to the sample receiving pad of the LFA and allow to absorb and migrate through the lateral flow assay. DNA detection on the LFA gives a final result in 10 minutes as detained under **Fig. 3B** **and** **C**.

Detection is rendered possible due to the labelling of the primers used for the amplification: C-allelic variant of -13910C/T primers or C-ASO primers) are labelled with digoxigenin which will be used as label for the amplified DNA first allele marker and T-allelic variant of - 13910C/T primers or T-ASO primers are labelled with Fluorescein isothiocyanate (FITC) or Fluorescein amidites (FAM)) biotin which will be used as label for the amplified DNA second allele marker. The labelled amplicon will be then detected by the antibodies specific to the label (anti-DIG and anti-FITC or anti-FAM) spotted onto the detection pad (test lines 1 and 2) of the lateral flow device and the biotin labelled part of the amplicon allows the visual detection of captured DNA with streptavidin labelled detection nanoparticles as detection reagent. A positive signal indicates that the DNA amplified contains C-allele (colored line T1) or T-allele (colored line T2) of -13910C/T polymorphism. The full genotype (-13910 C/T) of a sample can be determined (C/C, C/T or T/T). A negative result in line T1 indicates no amplification due to the absence of C-allele (C/C) only and is specific for a lactase non-persistence (lactose intolerance). A positive result in line T2 indicates the presence of T-allele (T/T). In case of both lines are positive (T1 and T2), it indicates the presence of both the C- and T-allele and is specific for a heterozygote polymorphism (C/T). A negative result in T2 indicates the absence of T-allele. If T2 is negative and T1 is positive, the test is specific for a homozygote polymorphism C/C (lactose intolerance). If both test lines (T1 and T2) are absent in the assay, it represents an invalid result because the only possible -13910 polymorphisms are: C/C, C/T or T/T (T or C allele are, at least, present once).

A first sets of experiments were carried out using as test sample in a method of the invention human genomic DNA obtained from a commercial source (Promega) defined as positive control which represents a DNA pool of several individuals expected to contain both the - 13910T and -13910C alleles. In fact, the method of the invention confirmed the presence of both -13910T/C alleles (**Fig. 4A**). Negative controls containing only primers without DNA yielded no signal.

Those data support that LFA sample receiving pads can be loaded with each individual LAMP reaction products for the -13910T and -13910C alleles (either T-ASO or C-ASO) or, alternatively, with pooled parallel LAMP reactions for the -13910T and -13910C alleles (T-ASO + C-ASO). To further validate these results, all LAMP reactions were double checked by agarose gel electrophoresis (lower panel in **Fig. 4A**). The typical multimer amplicons patterns are visible in the amplifications containing DNA, but no amplification bands were present on the NO-DNA controls.

A second set of experiments was carried out using as a target human genomic DNA obtained from 5 subjects in a method of the invention. The DNA was extracted using a commercially available method (NucleoSpin Tissue, Marcherey-Nagel) LAMP followed by LFA detection for all the 5 samples is shown in **Fig. 4B****.** Controls containing only primers in the absence of DNA yielded no signal. Based on these results, a genotype could be assigned to all samples analyzed in **Fig. 4B** (**1:** T/C, **2:** T/C; **3 and 4:** T/T and 5: T/C).

These results support that the primers of the invention (T-ASO primers) in combination with C-ASO primers allow to provide a full genotyping of the sample (C/C, C/T or T/T) when samples are run in parallel (C-allele and T-allele (of -13910C/T) amplified in parallel) even when using commercial DNA extraction and amplification kits and provided a validation of the advantages of those primers in a method of the invention.

### Example 2: Optimization of DNA extraction and amplification

The primers of the invention were used to develop a method of the invention were the extraction and amplification steps have been optimized.

### a1) Providing a biological sample obtainedfrom a subject

A sample from buccal cells with a swab or from a finger prick whole blood (e,g. < 50 µl) is collected. The patient must not eat, drink or smoke about 2 hours before the DNA sampling.

### a2) Extracting DNA from sample

The cells are then lysed in a lysis/extraction buffer comprising 25 mM NaOH + 0.2 mM EDTA in demineralized Water and sterile filtered (0.2 µm) using commercially available sterile filter unit. Blood sample is diluted 1:50 in an extraction buffer, corresponding to 15 µl of blood in 735 µl of extraction buffer. Saliva swab is mixed in 1 ml extraction buffer. The sample is then heated at 98°C for about 5 min and let cool down at room temperature or alternatively, the DNA is extracted at room temperature (e.g. 5 min) or could be extracted under heating, if needed.

### a3) DNA amplification

The isolated DNA is amplified using LAMP technology (LavaLAMP, Lucigen) with primers specifically designed to amplify only T allele (C/T or T/T) of -13910C/T polymorphism, specific for lactase persistence and labelled for the later detection of the amplified DNA in a rapid LFA. In particular, primers are labeled with Fluorescein amidite (FAM) or Digoxigenin (DIG) and biotin. The amplification step employs 5 template specific primers, of which one is an Allele Specific Oligo (ASO) primer, capable of recognizing -13910 T allelic form (T-ASO primer). The last primer (LB) is biotinylated and is used for the detection of amplicon on lateral flow assay as described in Example 1.

**For amplifying and labelling T allelic variant of -13910C/T polymorphism the following LAMP primers were used:**
- **The Forward Inner Primer (FIP)** consists of a F2 region at the 3' end and a F1c region at the 5' end. The FIP sequence used is of **SEQ ID NO: 1** with a FAM or DIG label on the F1c part (5' end of the primer).
- **The Forward Outer Primer (FOP, also called F3 primer)** consists of a region which is complementary to the F3c region of the template sequence. This primer is shorter in length and lower in concentration than FIP. The F3 sequence **(mutant type, T-allele)** used is of **SEQ ID NO: 2,** called T-ASO primer and corresponds to the DNA sequence just before the SNP of interest.
- **The Backward Inner Primer (BIP)** consists of a B2 region at the end of the 3'end and B1c region at the 5'end. The BIP sequence used is of **SEQ ID NO: 3.** as described in Example 1. The BIP could also be labelled (on the B1c part) with the same label used for FIP but only if the FIP is not labelled.
- **The Backward Outer Primer (BOP also called B3).** The BOP sequence used is of **SEQ ID NO: 4.**
- **The biotin labelled primer (LB).** The LB sequence used is of **SEQ ID NO: 5** which corresponds to a DNA sequence between the B1c and B2 with a biotin label at the 5' end.

**For amplifying and labelling A-allelic variant of -22018 G/A polymorphism the LAMP primers as described in Table 4 were used:**

**Table 4**

| **Primer name** | **DNA sequence (5'-3')** |
|---|---|
| **-22018 SNP** | |
| 22018-F3(A) | CCT TAA AAA CAG CAT TCT CAG CTG GGC A |
| 22018-FIP-FAM | (FAM) CAG GCT GGT CTC GAA CTC CTG ACC TCA GTG GCT CAC ACC TTT GTC |
| 22018-BIP | CCA ACA TGG CGA AAA CCC ATT CTG GGA CCA CAA GCA CCC GC |
| 22018-B3 | CCT GGG CTC AGT GAT CCT CC |
| 22018-LB-Bio | (Biotin)TAA AAA TAC AAA AAT TAG CC |

- **The Forward Inner Primer (FIP)** sequence used is as follows: caggctggtctcgaactcctgacctcagtggctcacacctttgtc **(SEQ ID NO: 6)** with a FAM label on the F1c part);
- **The Forward Outer Primer (FOP, also called F3 primer)** sequence **(A-allele)** used is as follows: ccttaaaaacagcattctcagctgggca **(SEQ ID NO: 7);**
- **The Backward Inner Primer (BIP)** sequence used is as follows: ccaacatggcgaaaacccattctgggaccacaagcacccgc **(SEQ ID NO: 8);**
- **The Backward Outer Primer (BOP also called B3)** sequence used is as follows: cctgggctcagtgatcctcc **(SEQ ID NO: 9);**
- **The [please complete] (LB)** sequence used is as follows: taaaaatacaaaaattagcc **(SEQ ID NO: 10)** with a biotin label.

A primer mix (10x concentrated) was prepared for each allele to be detected in 2 distinct tubes (one for 13910 and one for 22018). by mixing the following primers in a total volume of 100 µl, completed with sterile water:
- 2 µl of F3 primer (conc. 100 µM)
- 2 µl of B3 primer (conc. 100 µM)
- 10 µl of FIP primer (conc. 100 µM)
- 10 µl of BIP primer (conc. 100 µM)
- 10 µl of LB primer (conc. 100 µM)

After, both primer mixes are used in the DNA amplification buffer (according to Table 2 above).

Final concentration of the primer mix (10x concentrated) was 2 µM of F3 and B3 primers and 10 µM of FIP/BIP and LB primers. The primer mix was then diluted 10x for the LAMP reaction with a final concentration in the LA

MP reaction of 0.2 µM for F3/B3 and 1.0 µM for BIP/FIP and LB.

A LAMP kit such as described above was used to conduct LAMP amplification as described in Example 1 during 20-60 minutes at 72°C either with the set of primers for amplifying only T containing allele (TT or CT) or only A-containing allele (AA or AG) separately and combined.

### b) Amplified DNA detection

The obtained amplified DNA (amplicons) are diluted and detected by a nucleic acid lateral flow assay (LFA) incorporating antibodies which capture and allow visualization of doublestranded amplification products containing selected binding partners, FAM and biotin, as well as DIG and biotin. The amplicon detection takes only 5-10 minutes, and the result is detectable by eye due to an aggregation of detection reagent particles at the capture lines as detailed in Example 1 and below:

### Running the LFA assay

3 to 4 drops of the amplified DNA (e.g. about 200 µl) are diluted in a running buffer (Phosphate Buffered Saline solution, with 1-2% tween 20). A blocking agent such as BSA or other protein could be added to the running buffer to reduce non-specific binding.

The diluted amplified DNA is then added to the sample receiving pad of the LFA and allow to absorb and migrate through the lateral flow assay. DNA detection on the LFA gives a final result in 10 minutes as detained under **Fig. 3B** **and** **C****.**

Detection is rendered possible due to the labelling of primers used for the amplification, T-allelic variant of -13910C/T primers (T-ASO primers) is labelled with DIG and biotin **(****Fig. 2C** **and** **3Ba****).** The labelled amplicon will be detected by the antibodies specific to the label (anti-DIG) spotted onto the detection pad (reagent test line 1) of the lateral flow device and the biotin labelled part of the amplicon allows the visual detection of captured DNA with streptavidin labelled detection nanoparticles as detection reagent. A positive signal indicates that the DNA amplified contains T-allele (colored line T1) of -13910C/T polymorphism (T/T or T/C) and is characteristics for a lactase persistence. A negative result in line T1 indicates no amplification due to the absence of T-allele (genotype C/C) and is specific for lactase non-persistence called lactose intolerance.

The combination of -13910C/T primers showed a good detection performance (Test line 1, corresponding to antibody anti-DIG). The sample contains T-allele, either T/T or C/T, specific for lactose tolerance. Previous characterization of this sample showed that the positive sample has a C/T genotype.

Those data support that developed DNA extraction method of the invention allows an accurate, quick (<5-10 min) and easy determination of lactose intolerance using the quick extraction method, the LAMP amplification and LFA amplified DNA detection.

### List of elements referenced in the figures

1 Lateral flow immunoassay
2 backing support
3 capillary flow array
   5 sample receiving pad
   6 conjugate release pad
      61 detection reagent
         611a first binder to analyte
         611b first label moiety
      62 detection reagent support
   7 Detection pad
      71 capture test reagent array
         711a test reagent (antibody against the label of the analyte)
         711b optional further test reagent (e.g. another antibody against another label of the analyte)
      72 capture control reagent array
         721a control agent specific to first binder (e.g. specific antibody for the detection reagent)
      73 detection membrane
4 wicking pad

### SEQUENCE LISTING

***Primer sequences for LAMP of T allelic variant of -13910C*/*T polymorphism (5'-3' DNA sequence)***
   **SEQ ID NO: 1 - unlabeled Forward Inner Primer (FIP) sequence** tgcagggctcaaagaacaatctaactggcctcaaaggaactc
   **SEQ ID NO: 2- Forward Outer Primer (FOP or F3 primer)** ggcaatacagataagataatgtagt
   **SEQ ID NO: 3 - unlabeled Backward Inner Primer (BIP)** tccacgaggataggtcagtggaagatgggacgcttgaa
   **SEQ ID NO: 4 - Backward Outer Primer (BOP or B3)** taaaactaggaaaacgcagg
   **SEQ ID NO: 5 -biotin labelled (5' end) primer (LB)** ggggagtagtacgaaaggg
***Primer sequences for LAMP of A allelic variant of -22018G*/*A polymorphism (5'-3' DNA sequence)***
   **SEQ ID NO: 6 - unlabeled Forward Inner Primer (FIP) sequence** caggctggtctcgaactcctgacctcagtggctcacacctttgtc
   **SEQ ID NO: 7 - Forward Outer Primer (FOP or F3 primer)** ccttaaaaacagcattctcagctgggca
   **SEQ ID NO: 8 - unlabeled Backward Inner Primer (BIP)** ccaacatggcgaaaacccattctgggaccacaagcacccgc
   **SEQ ID NO: 9 - Backward Outer Primer (BOP or B3)** cctgggctcagtgatcctcc
   **SEQ ID NO: 10 - biotin labelled (5' end) primer (LB)** taaaaatacaaaaattagcc
***Primer sequences for LAMP of C allelic variant of -13910C*/*T polymorphism (5'-3' DNA sequence)***
   **SEQ ID NO: 11 - Forward Outer Primer (FOP or F3 primer)** ggcaatacagataagataatgtatc

## Claims

1. A lateral flow immunoassay device for qualitative or quantitative detection of the presence of a T allele (-13910 C/T) or A allele (-22018 G/A) in a biological sample comprising a backing support and, on said backing support, a capillary flow array and a wicking pad, wherein the capillary flow array comprises **i)** a sample receiving pad located at one end of the backing support and having pores so as to receive the sample; **ii)** a conjugate release pad being distinct from the sample receiving pad or included to the sample receiving pad and being in capillary contact with the sample receiving pad and being impregnated with at least one detection reagent comprising a first binder specific and binding to at least one labelled allele selected from T allele (-13910 C/T) or A allele (-22018 G/A) analyte conjugated to a first label moiety; **iii)** a detection pad being distinct from the conjugate release pad and being in capillary contact with the conjugate release pad and comprising a detection membrane, a capture test reagent array comprising at least one reagent test line and a capture control reagent array comprising one control reagent line, said capture reagent and control reagent arrays being immobilized on the detection membrane and **iv)** a wicking membrane at the other end of the backing support and being in capillary contact with the detection pad, wherein said at least one reagent test line comprises at least one antibody against the label of said labelled allele and said control reagent line comprises a control reagent having a specific affinity for the first binder.

2. A lateral flow immunoassay device according to claim 1 wherein the conjugate release pad comprises a detection reagent support wherein the said at least said one detection reagent is bound in a capillary releasable manner, receives the sample from the sample receiving pad and releases the detection reagent from its detection reagent support and a first immuno-complex is formed when the labelled allele selected from T allele (-13910 C/T) or A allele (-22018 G/A analyte is present in the sample and is combined with the detection reagent, the said first immuno-complex then migrates to the detection pad where it binds to the said at least one reagent test line comprising the antibody against the label of said labelled allele.

3. A lateral flow device according to claim 1 or 2 wherein the detection pad further comprises more than one reagent test line in the capture test reagent array.

4. A lateral flow device according to any one of claims 1 to 3 wherein the capture test reagent array comprises at least one antibody selected from an anti-DIG antibody, an anti-FAM and/or an anti-FITC antibody or a combination thereof.

5. A lateral flow immunoassay according to any one of claims 1 to 4, wherein the detection reagent comprises streptavidin protein as first binder to the analyte.

6. A method for detecting the presence of T allele (-13910 C/T) and/or of A allele (-22018 G/A) in a sample, said method comprising:
a) Providing a sample comprising DNA material (amplicons) in aqueous solution, wherein in said DNA material has been subjected to specific amplification and labelling targeting DNA from T allele (-13910 C/T) and/or of A allele (-22018 G/A);
b) Subjecting the said sample to a lateral flow immunoassay comprising **i)** a conjugate release pad comprising detection reagents for said labelled amplified alleles and **ii)** a detection pad comprising a detection membrane where at least one antibody against the label of said amplified allele(s) is bound;
c) Detecting the presence or absence of a detection line on the detection membrane at the location where the said at least one antibody against the label of said amplified allele(s) is bound, the presence of said detection line being indicative of T allele (-13910 C/T) and/or of A allele (-22018 G/A) in said sample.

7. A method according to claim 6 wherein at least one anti-DG antibody or a mixture thereof is further bound to the detection membrane of said lateral flow immunoassay and the detection of the presence of DG labelled T allele (-13910 C/T) is carried out under step c).

8. A method according to claim 6 or 7 wherein at least one anti-FAM or anti-FITC antibody or a combination of those, is bound to the detection membrane of said lateral flow immunoassay and the detection of the presence of FAM and/or FITC A allele (-22018 G/A) is carried out under step c).

9. A method according to any ones of claims 6 to 8, wherein the absence of a T allele (-13910 C/T) and/or of A allele (-22018 G/A) is indicative of lactose intolerance.

10. A method according to any ones of claims 6 to 9, wherein the DNA material provided under step a) has been obtained by LAMP amplification of DNA extracted from a biological sample before LAMP amplification in an extraction step where the said biological sample is subjected to a DNA extraction step in presence of an extraction buffer comprising from about 0.1 to about 0.5 mM EDTA and from about 20 to about 50 mM NaOH.

11. A method according to any ones of claims 6 to 10, wherein the amplified DNA material (amplicons) is obtained from LAMP amplification wherein DNA from T allele (-13910 C/T) is specifically amplified and labelled using a set of the following primers selected from the following group:
- **a FIP primer** comprising a sequence of **SEQ ID NO: 1;**
- **a FOP or F3 primer** comprising a sequence of **SEQ ID NO: 2;**
- **a BIP primer** comprising a sequence of **SEQ ID NO: 3;**
- **a BOP or B3 primer** comprising a sequence of **SEQ ID NO: 4;**
- **a LB primer** for labelling the resulting amplified DNA with biotin, wherein the resulting labeled amplified DNA can be detected by the detection reagent of a lateral flow assay; and wherein either the FIP or the BIP primer is labelled with a labelling group for immune or fluorescence detection such as a Fluorescein amidite (FAM) or Fluorescein isothiocyanate (FITC) label or for immune detection such as Digoxigenin (DIG) label.

12. A method according to claim 11, wherein the LB primer used for LAMP amplification of DNA from T allele (-13910 C/T) comprises a sequence of **SEQ ID NO: 5.**

13. A method according to any one of claims 6 to 12, wherein the amplified DNA material (amplicons) is obtained from LAMP amplification wherein DNA from A allele (-22018G/A) is specifically amplified and labelled using a set of the following primers selected from the following group:
- **a FIP primer** comprising a sequence of **SEQ ID NO: 6;**
- **a FOP or F3 primer** comprising a sequence of **SEQ ID NO: 7;**
- **a BIP primer** comprising a sequence of **SEQ ID NO: 8;**
- **a BOP or B3 primer** comprising a sequence of **SEQ ID NO: 9;**
- **a LB primer** for labelling the resulting amplified DNA with biotin, wherein the resulting labeled amplified DNA can be detected by the detection reagent of a lateral flow assay; and wherein either the FIP or the BIP primer is labelled with a labelling group for immune or fluorescence detection such as a Fluorescein amidite (FAM) or Fluorescein isothiocyanate (FITC) label or for immune detection such as Digoxigenin (DIG) label.

14. A method according to claim 13, wherein the LB primer used for LAMP amplification of DNA from A allele (-22018G/A) comprises a sequence of **SEQ ID NO: 10.**

15. A LAMP kit for amplification and labelling DNA from T allele (-13910 C/T) and/or of A allele (-22018 G/A), said LAMP kit comprising the following set of primers: **a FIP primer** comprising a sequence of **SEQ ID NO: 1;**
- **a FOP or F3 primer** comprising a sequence of **SEQ ID NO: 2;**
- **a BIP primer** comprising a sequence of **SEQ ID NO: 3;**
- **a BOP or B3 primer** comprising a sequence of **SEQ ID NO: 4;**
- **a LB primer** for labelling the resulting amplified DNA with biotin, wherein the resulting labeled amplified DNA can be detected by the detection reagent of a lateral flow assay; and wherein either the FIP or the BIP primer is labelled with a labelling group for immune or fluorescence detection such as a Fluorescein amidite (FAM) or Fluorescein isothiocyanate (FITC) label or for immune detection such as Digoxigenin (DIG) label;
and/or
- **a FIP primer** comprising a sequence of **SEQ ID NO: 6;**
- **a FOP or F3 primer** comprising a sequence of **SEQ ID NO: 7;**
- **a BIP primer** comprising a sequence of **SEQ ID NO: 8;**
- **a BOP or B3 primer** comprising a sequence of **SEQ ID NO: 9;**
- **a LB primer** for labelling the resulting amplified DNA with biotin, wherein the resulting labeled amplified DNA can be detected by the detection reagent of a lateral flow assay; and wherein either the FIP or the BIP primer is labelled with a labelling group for immune fluorescence detection such as a Fluorescein amidite (FAM) or Fluorescein isothiocyanate (FITC) label or for immune detection such as Digoxigenin (DIG) label.

16. A kit for qualitative detection of lactose intolerance in a biological sample, said kit comprising at least one lateral flow immunoassay device according to any one of claims 1 to 5 or at least one primer or set of primers as described in claim 15.

17. A kit according to claim 16 further comprising at least one of the following:
- a sample collection device;
- a container with a buffering solution for dilution of the sample or for running the test with pure sample;
- a disinfection pad;
- an adhesive plaster;
- a data reader for reading the assay results and optionally transmitting to a database (e.g. patient hospitalization data set);
- a portable heater to heat the LAMP reaction medium at 70-80°C.
